# EUROPEAN PATENT APPLICATION

(11) **EP 1 550 658 A1**
(43) Date of publication of application: **06.07.2005**
(21) Application number: 04258181.9
(22) Date of filing: 30.12.2004
(51) Int. Cl.: C07D 261/08, A61K 31/42

(54) **Method for preparing 3,4-diphenyl-substituted isoxazole compounds**

(30) Priority: 28.07.2004 US 591799 P; 30.12.2003 IN ch10632003; 21.12.2004 US 637782 P
(71) Applicant: Dr. Reddy's Laboratories Ltd., Hyderabad 500 016 (IN); Dr. Reddy's Laboratories, Inc., Bridgewater, NJ 08807 (US)
(72) Inventor: Sundaram, Venkataraman, Moti Nagar, Hyderabad 500 018 (IN); Bhimireddy, Anuradha, Khammam Dist. 507 164, Andhra Pradesh (IN); Eswaraiah, Sajja, Hyderabad 500 072, Andhra Pradesh (IN); Goverdhan, Gilla, Gayatri Residency, Hyderabad 500 050, Andhra Pradesh (IN); Purandhar, Koilkonda, Hyderabad 500 072, Andhra Pradesh (IN); Rajendra, Surasani, Hyderabad 500 072, Andhra Pradesh (IN); Raju, Nadimpally Satyavarahala, Hyderabad 500 033, Andhra Pradesh (IN); Reddy, Anumula Raghupathi, Hyderabad 500 072, Andhra Pradesh (IN); Reddy, Lekkala Amarnath, Hyderabad 500 060, Andhra Pradesh (IN); Reddy, Emani Srinivasa, Hyderabad 500 072, Andhra Pradesh (IN); Reddy, Padi Prathap, H. No. 4-7-17/5/2, Plot No., Hyderabad 500 076, Andhra Pradesh (IN); Sampath, Alla, Hyderabad 500 072, Andhra Pradesh (IN); Sravanthi, Vecha, Khammam 500 007, Andhra Pradesh (IN)
(74) Representative: Bates, Philip Ian

(57) **Abstract**

The present invention relates to a process for preparing diaryl-substituted isoxazole using compounds of Formula (V) and Formula (VII): 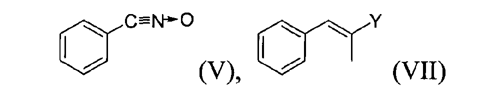 where Y is 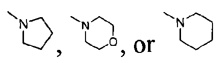 and to processes for preparing valdecoxib and parecoxib.

## Description

The present application claims benefit of a filing date of an Indian Patent Application No. 1063/CHE/2003, filed December 30, 2003, the contents of which are expressly incorporated herein by reference and filing dates of U.S. provisional application 60/591,799 filed July 28, 2004 and U.S. provisional application (application number is not available) with the title of "Method for preparing benzenesulfonyl compounds" filed December 21, 2004, of which the entire contents are expressly incorporated herein by reference.

### BACKGROUND

The present invention relates to a process for the preparation of diaryl-substituted isoxazole compounds such as valdecoxib, parecoxib, and their intermediates.

Diaryl-substituted isoxazole compounds are known selective COX-2 inhibitors. For example, 4-(5 Methyl-3-phenyl-4 - isoxazolyl) benzene sulfonamide (also known as Valdecoxib) is used for the treatment of rheumatoid arthritis, osteoarthritis, and the dysmenorrheapain. In addition sodium salt of N-[[(5 Methyl-3-phenylisoxazol-4-yl) phenyl] sulfonyl] propanamide (also known as Parecoxib) is particularly effective in parenteral compositions for acute pain management.

US patent publication 2003/0162813 discloses a process for preparing valdecoxib, which comprises the reaction of 4-acetyl benzene sulfonyl chloride with ammonium hydroxide in ether gives 4-sulfonyl acetopheneone, which is reacted with lithium diisopropylamide and hexamethyl phosphoramide to yield 1-(4-sulfonyl phenyl)-propyne. Then, this propyne compound is reacted with benzonitrile oxide in ethanol at reflux temperature to give valdecoxib. However, this process is very disadvantageous for commercial scale production of the compound due to moisture sensitive reagents like lithium agents and to poor yields.

### SUMMARY OF THE INVENTION

In accordance with one aspect, the invention provides a process for a diaryl-substituted isoxazole of Formula (IX):

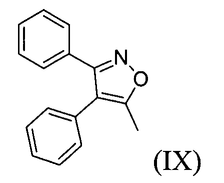

The process includes a condensation reaction of a compound of Formula V with a compound of Formula (VII): 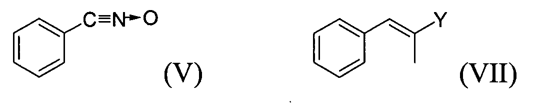 wherein Y is 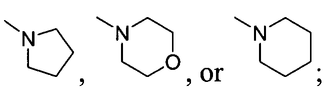 and removal of Y group after the condensation reaction.

In other aspect, the invention also provides a process for the preparation of diaryl-substituted isoxazole of Formula I: 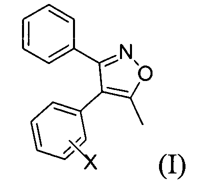 wherein X is R-SO₂-, in which R is C₁-C₆ alkyl, C₁-C₆ alkanoylamino and amino. The process involves a condensation reaction between Formulas (V) and (VII); removal of Y group after the condensation reaction; a chlorosulfonation with ClSO₃H; and a substitution of the chloro group with the R group.

Yet, in another aspect of the invention, processes for preparing valdecoxib and parecoxib are also provided.

### DETAILED DESCRIPTION

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention the preferred methods and materials are described.

Unless stated to the contrary, any use of the words such as "including," "containing," "comprising," "having" and the like, means "including without limitation" and shall not be construed to limit any general statement that it follows to the specific or similar items or matters immediately following it. Except where the context indicates to the contrary, all exemplary values are intended to be fictitious, unrelated to actual entities and are use for purposes of illustration only. Most of the foregoing alternative embodiments are not mutually exclusive, but may be implemented in various combinations. As these and other variations and combinations of the features discussed above can be utilized without departing from the invention as defined by the claims, the foregoing description of the embodiments should be taken by way of illustration rather than by way of limitation of the invention as define by the appended claims.

The process for preparing the compound of Formula (IV) is schematically represented in Scheme I.

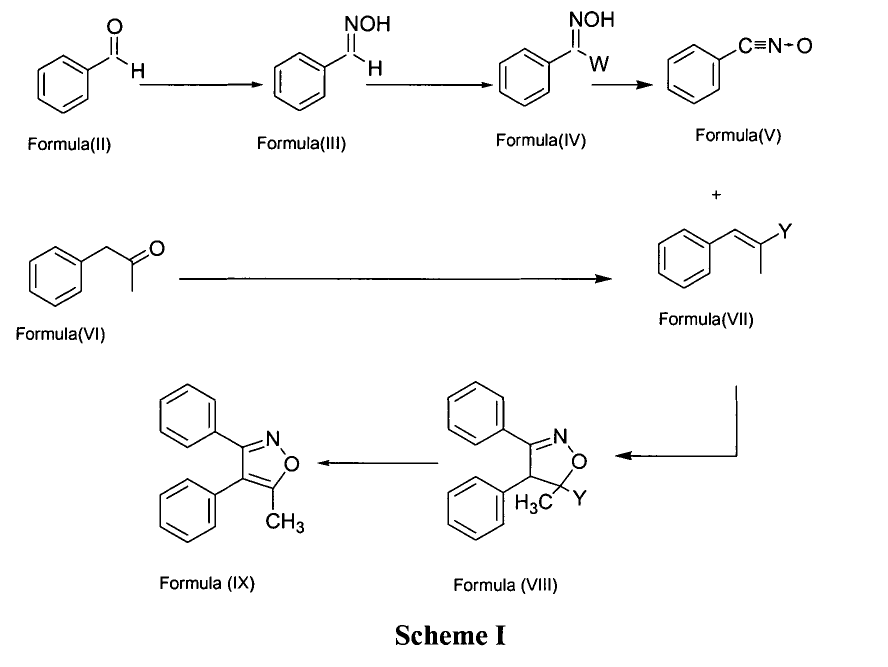

The compound of Formula (V) can be prepared from phenylaldehyde of Formula (II) as shown in Scheme I.

Preparation of the compound of Formula (III) involves a condensation of the compound of Formula (II) with hydroxylamine hydrochloride in the presence of a suitable base, such as sodium hydroxide, potassium hydroxide, sodium acetate and like in a suitable solvent, such as water, C₁-C₄ alcohol, and like. The reaction temperature ranges from about 25°C to reflux temperature of the solvent used, preferably about 25 - about 35°C. The reagents used in the process are molar excess and may range from about 1 mole to about 3 mole ratio.

Transformation of the compound of Formula (III) into the compound of Formula (IV) involves allylic halogenation. The suitable allylic halogenating reagents like N-chloro succinimide, N-bromo succinimide, dibromo dimethylhydantoin and like with or without suitable free radical initiators such as 2,2¹-azobisisobutyronitrile and dibenzoyl peroxide. A suitable solvent for the reaction may include dimethyl formamide, carbon tetrachloride, chloroform, dichloro methane, cyclohexane and like.

The reagents in the above process may be used in about equimolar amount or in about one or two molar excess. The temperature may ranges from about 25°C to reflux temperature of the solvent used.

Then, the compound of Formula (IV) can be converted to the compound of Formula (V) by dehydrohalogenation in the presence of a suitable base such as triethylamine, pyridine, pyrrolidine, sodium ethoxide, sodium methoxide and like in a suitable solvent such as toluene, benzene, chloroform, dichloromethane, cyclohexane and like. The temperature ranges from about 5°C to about 35°C, preferably about 5 - about 10°C. The reagents used in the process may range from about equimolar to about 2 mole ratio.

The compound of Formula (VII) can be prepared by condensation of the compound of Formula (VI) wherein Y is pyrrolidine, morpholine or piperidine in the presence of a suitable dehydrating reagent, such as potassium carbonate, sodium carbonate, sodium sulfate, magnesium sulfate, calcium chloride and like in a suitable solvent such as diethyl ether, diisopropyl ether, tetrahydrofuran, methyl tert. butyl ether, C₁- C₄ alcohol, toluene, benzene and like. The temperature ranges from about 10°C to reflux temperature of the solvent used, preferably about 25 - about 35°C. The reagents used in the above process range from about 0.9 moles to about 3 mole ratio.

The condensation of the compounds of Formula (V) and Formula (VII) may be performed in a suitable solvent, such as toluene, benzene, chloroform, dichloromethane, CCl₄, C₁-C₄ alcohol and like. The reaction temperature may range from about 5°C to reflux temperature of the solvent used, preferably between about 25 and about 35°C.

The compound of Formula (IX) may be prepared by de-amination with or without isolation of Formula (VIII) in the presence of a suitable acid like hydrochloric acid, hydrobromic acid, sulfuric acid and like in suitable solvent, such as water, toluene, benzene, C₁-C₄ alcohol and like. The temperature may range from about 25°C to reflux temperature of the solvent used. The reagents used in the process may range from about equimolar to about 7 mole ratio, preferably about 5- about 6 mole ratio.

The compound of Formula (IX) prepared according to the above described process can be used to prepare the compound of Formula (I): 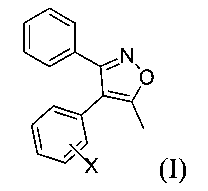 wherein X is R-SO₂-, in which R is C₁-C₆ alkyl, C₁-C₆ alkanoylamino and amino.

The sulfonation of the compound of Formula (IX) may be achieved by using chlorosulfonic acid. Chlorosulfonation is done in a suitable solvent such as dichloro methane, chloroform, cyclohexane or neat. The temperature may range from about 0°C to reflux temperature of the solvent used. Then, the chloro group can be substituted with R group. When R group is amino group, the amination may be carried out using ammonium hydroxide in suitable solvent such as dichloromethane, chloroform, toluene, benzene, cyclohexane and like. The temperature may range from about 0°C to reflux temperature of the solvent used, preferably about 0°C to about 35°C. The reagents used in the process are molar excess and may range from about 2 moles to about 12 mole ratio. The X group may be attached at the *ortho, meta* or *para* position, preferably at the *meta* or *para* position, even more preferably at the *para* position.

When R is amino group and X is at the *para* position, the compound of Formula (I) would have a structure of Formula (Ia): 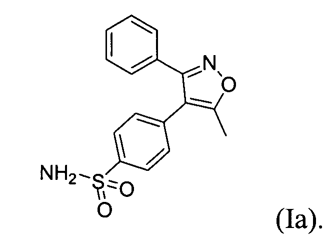 The compound of Formula (Ia) is valdecoxib.

The compound of Formula (Ia) can be converted to sulfonyl amide compound using anhydride compounds. For example, a reaction between valdecoxib with propionic anhydride would provide N-[[4-(5-methyl-3-phenyl-4-isoxazolyl)phenyl]sulfonyl] propanamide, which is also known as parecoxib of Formula (Ib).

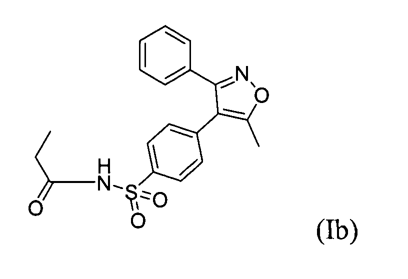

The compound of Formula (Ib) may be further converted without isolation to its salt using an alkaline base such sodium hydroxide, potassium hydroxide, sodium alkoxide, sodium hydride, sodium carbonate, etc.

In an alternative procedure, the chloro sulfonated compound of Formula (X) can be directly converted to the sulfonyl amide compounds such as parecoxib using alkanyl amide such as methyl amide, ethyl amide, propanyl amide, etc.

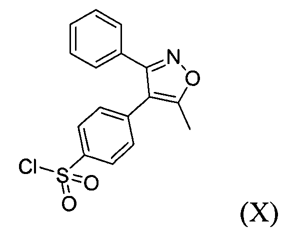

In case of parecoxib, the benzenesulfonyl chloride compound (X) is reacted with propanamide under acidic condition to give parecoxib.

The process described in the present invention is demonstrated in examples illustrated below. These examples are provided as illustration only and therefore should not be construed as limitation of the scope of invention.

### EXAMPLE: 1

### PREPARATION OF BENZALDEHYDE OXIME (III)

Solution of 32.5kg of hydroxylamine hydrochloride in 90 lit. of water in small portions were added to the mixture of 68 lit. of water, 46kg of sodium hydroxide and 45 kg of benzaldehyde at 25-35°C, and the reaction mass was maintained at 25-35°C for 30-45 minutes. The pH of resulted reaction mixture was adjusted to 6.5-7.5 with 7-15 lit. of hydrochloric acid and extracted with 225 lit. methylene chloride in three lots. The total organic layer was charged into reactor and methylene chloride was distilled off below 50°C under atmospheric pressure and again distilled off methylene chloride under vacuum until temperature reaches to 65-70°C. The reaction mass was maintained at 65-70°C under vacuum for 1-2 hours. It was then cooled to 20-30°C.The yield of the title compound is 49 kg. (95%).

### EXAMPLE: 2

### PREPARATION OF CHLORO BENZALDEHYDE OXIME (IV)

240 lit. of dimethylformamide and 63.4 kg of N-chloro succinimide were added into reactor. To the above solution, 48 kg of benzaldehyde oxime in 48 lit. of dimethylformamide was added slowly at 25-35°C. The resulting reaction mixture was stirred at 25-35°C for 1-1.5 hours and poured into 720 lit. of water slowly at 25-35°C, which was stirred for 30-45 minutes and extracted with 335 lit. of methylene chloride in three lots. The total organic layer was washed with solution of 4.8 kg of hydrose in 95 lit. of water followed by 190 Lts. of water twice. Methylene chloride was concentrated under atmosphere pressure at 45- 50°C followed by vacuum at 45-50°C for 1 and 11/2 hrs and cooled the residue to 25-35°C. The resultant residue is used at next stage directly as a part of in situ reaction.

### EXAMPLE: 3

### PREPARATION OF 1-(1-METHYL-2-PHENYL ETHYNYL) PYRROLIDINE (VII)

370 lit. of cyclohexane, 4.25 kg of pyrrolidine and 45.2 kg of phenyl acetone were charged into a reactor, and the reaction mass was heated to azeotropic reflux at 72-82°C upto water collection stopped. It was then cooled to 40-50°C and distilled the solvent completely under vacuum until temperature reaches to 45-50°C under vacuum for 1.5-2 hrs and again cooled the residue to 25-35°C to get title compound.

### EXAMPLE: 4

### PREPARATION OF 3,4 - DIPHENYL-5-METHYL -5-PYRROLIDINYL ISOXAZOLINE (VIII)

Residue containing chlorobenzaldehyde was dissolved in 95.1lit. of methylene chloride and allowed to cool to 5-10°C. The solution was added to a mixture of 1-(1-Methyl-2-phenyl ethynyl) pyrrolidine, triethyl amine (53kg) and methylene chloride (5751it.), and the resulting reaction mixture was stirred for 10-15 minutes at 5-10°C. The temperature of reaction mass was raised to 25-35°C and stirred at the same temperature for 2-3 hours. Water (360 lit.) was added to the above reaction mixture and stirred for 15-30 minutes. The reaction mixture was allowed to settle for 20-30 minutes. Separated organic layer was added to a reactor and methylene chloride was distilled off form the organic layer under atmospheric pressure at 50-55°C to afford the residue of 3,4 - Diphenyl-5-methyl -5-pyrrolidinyl isoxazoline. The resultant residue is used at next stage directly.

### EXAMPLE: 5

### PREPARATION OF 3, 4 - DIPHENYL-5- METHYL ISOXAZOLE (IX)

480 lit. of water was added to residue of 3,4 - diphenyl-5-methyl-5-pyrrolidinyl isoxazoline. Then 226 lit. of hydrochloride acid was added into this mix at 40-50°C. The reaction mass was heated, and the distillate was collected until temperature reached to 90°C. The resulted mass was again refluxed at 98-102°C for 11/2 and 21/2 hours and allowed to cool to 25-35°C. The reaction mass was then extracted with 440 lit of methylene chloride in two lots. The organic layer was washed with 145 lit. of water. Methylene chloride was distilled off under atmosphere pressure followed by vacuum at 45-50°C for 1 and 11/2 hrs. Isopropyl alcohol (35 lit.) was added to the residue, and isopropyl alcohol was distilled off under vacuum until temperature reaches to 45-50°C for 1 and 11/2 hrs. 75 lit. of isopropyl alcohol was added again into the residue and stirred at 45-50°C for 15-30 mins. The resulted solution was cooled to 0-5°C, stirred for 45-60 minutes at 0-5°C and centrifuged. The resulted wet cake was washed with chilled isopropyl alcohol (3.15 lit) and was spin dried for 30-45 minutes. The resulted wet material was charged into reactor and heated reaction mass to 57.5±2.5°C minute till complete dissolution and cooled the reaction mass to 30±5°C for about 55±5 minute and centrifuged. The resulted wet cake was washed with isopropyl alcohol and was spin dried for 30-45 minutes and centrifuged. The resulted wet material was dried under vacuum for 30-45 minutes without hot water circulation followed by hot water circulation to the drier jacket at 40-45°C for 30-45 minutes to afford the title compound. (Yield 24 kg. 25.6%).

### EXAMPLE: 6

### PREPARATION OF 4-(5-METHYL-3-PHENYL-4-ISOXAZOLYL) BENZENE SULFONYL CHLORIDE (X)

75 lit. of methylene chloride was added into reactor and cooled to 10-15°C and added slowly 98.00 kg of chlorosulfonic acid. To this, solution of 3, 4 - Diphenyl-5- methyl isoxazole in 50 lit. of methylene chloride was added slowly to the reaction mass at 0-10°Cand heated to reflux for 9-11 hours. The reaction mass was cooled to 25-35°C. The resultant reaction mass was added slowly to the chilled water (175 lit.) at 0-10°C, and temperature of reaction mass was raised to 25-35 °C. Aqueous and organic layer were separated, and aqueous layer was extracted with 125 lit of methylene chloride in two lots. The combined organic layer was washed with 575 lit water in three lots. The combined organic layer was charged into a reactor and distilled of methylene chloride below 60°C. The resultant crude solid was dissolved in 250 lit. of cyclohexane and heated to reflux for 30-45 minutes. 75 lit. of water was added to the reaction mass and again heated to reflux for 15-30 minutes. The reaction mass was settled. Organic and bottom aqueous layer were separated. Organic layer was cooled to 25-35°C and maintain at 25-35°C for 45-60°C and filtered and washed with cyclohexane. This step was repeated twice. Finally the wet compound was dried at 50-55 °C to afford the title compound.(Yield 21.3kg, 59.15%).

### EXAMPLE: 7

### PREPARATION OF 4-(5-METHYL-3-PHENYL-4-ISOXAZOLYL) BENZENE SULFONAMIDE (Ia)

Charged 120 lit. of methylene chloride 20 kg of 4-(5-methyl-3-phenyl-4-isoxazolyl) benzene sulfonyl chloride into reactor and stirred the reaction mass for 5 to 10 minutes till the dissolution. 1 kg of carbon was charged into above solution, stirred for 30-45 minutes, filtered, and washed with 40ml of methylene chloride. The combined filtrate was charged into reactor and 90 lit. of ammonia solutions were added into the filtrate at 20-30°C for 60-90 minutes. Distilled methylene chloride at atmospheric pressure below 40°C upto 300 to 340 lit. volume left in the reactor. The reaction mass was cooled to 5-10°C and stirred for 30-45 minutes and filtered and washed with 20 lit. methylene chloride. The resultant wet cake was pressed under Nitrogen for 30-45 minutes. The wet material was charged into reactor and 100 lit. of water were added into reactor and stirred for 45-60 minutes and filtered, pressed the wet cake under nitrogen for 15-20 minutes followed by washing with 20 lit. of water. Again wet cake was pressed under nitrogen for 30-45 minutes. The resultant compound was dried under vacuum for one hours at 25-35 °C and further dried at 80-85 °C for 1 hours to afford the title compound.(Yield 15kg,80%).

### EXAMPLE: 8

### PREPARATION OF BENZALDEHYDE OXIME (III)

Hydroxylamine hydrochloride (72 g, 1.1 mol) was added in small portions to the mixture of 190 ml of water, 64 g (1.7 mol) of sodium hydroxide and 100 g (1.0 mol) of benzaldehyde. The reaction mass was stirred for 4-5 hours and diluted with 2400 ml of water. The resulted reaction mixture was neutralized with 44 ml of hydrochloric acid and extracted with 3 X 250 ml of ether. The organic layer was dried over 24 g of sodium sulfate and ether was distilled off below 45°C under reduced pressure. The yield of the title compound is 87.5 g (76.71%).

### EXAMPLE: 9

### PREPARATION OF CHLORO BENZALDEHYDE OXIME (IV)

One-tenth to one-fifth of 13.2 g (1.20 mol) of N-chloro succinimide was changed to the mixture of 69 ml of dimethyl formamide and 10 g (1.0 mol) of benzaldehyde oxime at 25-30°C. and then the remaining N-chloro succinimide was added to the reaction mass at below 35°C. The reaction mass was stirred for 60 minutes at 25-35°C and poured into 275 ml of water. The title compound was extracted with 2 X 27 ml of ether, washed with 3 X 27 ml of water and dried over 2.7 g of sodium sulfate. Ether layer was concentrated under reduced pressure. The yield of the obtained compound is 11.8 g (91.8 %).

### EXAMPLE: 10

### PREPARATION OF BENZONITRILE OXIDE (V)

Chlorobenzaldehyde oxime (15.0 g 1.0 mol) was dissolved in 290 ml of benzene. The resulted solution was cooled to 5-10°C with vigorous stirring and was charged with 13.5 ml (1.0 mol) of triethylamine. After stirring for 5-10 minutes, separated solid was removed by filtration with help of additional benzene (48 ml). The filtrate was taken for the preparation of compound of Formula (VIII).

### EXAMPLE: 11

### PREPARATION OF 1-(-METHYL-2-PHENYL ETHYNYL) PYRROLIDINE (VII)

Potassium carbonate was charged to the mixture of 188 ml of ether, 40.0 g (1.5mol) of pyrrolidine and 50.0 g (1.0 mol) of phenyl acetone. The resulted suspension was stirred for 3-4 days. The reaction mixture was filtered, and the solvent of the filtrate was evaporated. The residue was further dried at about 150-160 °C under high vacuum. The yield of the title compound is 49.9 g (71.6 %).

### EXAMPLE: 12

### PREPARTAION OF 3,4 - DIPHENYL-5-METHYL-5-PYRROLIDINYL ISOXAZOLINE (VIII)

The benzonitrile oxide (V) filtrate obtained in Example 3 was charged to the mixture of 54 ml of benzene and 20.0 g (1.0 mol) of 1-(1-methyl-2-phenyl ethenyl) pyrrolidine at 5-10°C. The temperature was raised to 25-35°C, and after stirred for 10 minutes, the reaction mixture was diluted with 100 ml of water. Benzene layer was separated and distilled below 60°C under reduced pressure. The residue was cooled to 25-35°C, and 20 ml of n-heptane was added. After stirred for 30- 45 minutes, the heptane mixture was filtered to isolate solid, which was washed the solid with 10 ml of n-heptane. The isolated compound was dried at 70 - 75°C. The yield of the product is 9.9 g (30 %). Mass Spectrometry Calculated for C₂₀H₂₂N₂O 307 (M⁺¹), found 307.

### EXAMPLE: 13

### PREPARATION OF 3, 4 - DIPHENYL-5-METHYL ISOXAZOLE (IX)

6N HCl (32.0 g) was charged to the suspension of 80 ml of water and 8.0 g of 3,4 - diphenyl-5-methyl-5-pyrrolidinyl isoxazoline. The reaction mass was heated to reflux for 2 hr, cooled to 25-35°C and charged with 24 ml of dichloromethane. Two layers were separated, and aqueous layer was extracted with 2x24 ml of dichloromethane. The combined dichloromethane layer was washed with 2x24 ml of 10% NaHCO₃ solution followed by 40 ml of water. The dichloromethane layer was dried over 8.0 g of Na₂SO₄ and concentrated under reduced pressure below 45°C. The residue was dried to 25-35°C and charged with 8.0 ml of isopropyl alcohol and 40.0 ml of n-heptane. The resulting solution was cooled to 0-5°C and stirred for 45-60 minutes. The precipitates were filtered, washed with 8.0 ml of n-heptane and dried at 50-55°C in vacuum oven. The yield of the compound is 3.4 g (55.4%).

### EXAMPLE: 14

### ONE POT PROCESS FOR THE PREPARATION OF 3, 4-DIPHENLY-5-METHYL ISOXAZOLE (IX)

Benzonitrile oxide (V) filtrate of Example 3 was charged to the mixture of 134 ml of benzene and 50 g (1.0 mol) of 1-(1-methyl-2- phenyl ethenyl) pyrrolidine(VII) at 5- 10°C. Reaction temperature was raised to 25-35°C, and the solution was stirred for 1 hour. The solution was diluted with 264 ml of water. Benzene layer was separated and distilled under reduced pressure below 60°C. Water (250 ml) and 322.2 ml of 6N HCl was added to the residue at 25-35°C. After heated to reflux for 5 hour, the resulting reaction mass was cooled to 25-35°C and diluted the reaction mass with 250 ml of dichloromethane. The dichloromethane layer was separated, and the aqueous layer was extracted with 150 ml of dichloromethane. The combined dichloromethane layers were washed with 2 X 200 ml of 10 % sodium bicarbonate solution and subsequently with 150 ml of saturated brine solution and with 150 ml of water. The dichloromethane layer was dried over 25 g of sodium sulfate, and the dichloromethane was removed completely below 45°C under reduced pressure. Isopropyl alcohol (25 ml) and 100 ml of n-heptane were added to the residue at 25-35°C, cooled to 0-5°C, stirred for 45-60 minutes at 0-5°C and filtered to isolate the solid compound which was, washed with 25 ml of n-heptane and dried the solid compound at 50-55°C under reduced pressure. The yield of the titled product is 14.0 g (22.2 %).

### EXAMPLE: 15

### PREPARATION OF 4-(5-METHYL-3-PHENYL-4-ISOXAZOYL) BENZENE SULFONAMIDE (Ia)

Chlorosulfonic acid (9.6 ml, 11.5 mol) was added slowly to 3.0 g (1.0 mol) of 3,4-dipheny-5-methyl isoxazole at 0-5°C. After a clear solution was obtained, slowly the solution was heated to 50-60°C and stirred for 2 hours. The solution was poured into a mixture of 130 ml of dichloromethane and 130 ml of chilled water. The dichloromethane layer was separated and cooled to 0-5°C. Ammonium hydroxide solution (65 ml) was added to the dichloromethane layer, which was stirred for 2 hours. The dichloromethane layer was separated, and the aqueous layer was extracted with 30 ml of dichloromethane. The combined dichloromethane layer dried over 1.0 g of sodium sulfate. The dichloromethane layer was condensed to approximately one-half of its original volume, cooled to 0-5°C and stirred for 30 minutes. Solid was separated by filtration and washed with 3.0 ml of chilled dichloromethane. The filtered compound was dried at 50-55°C under vacuum. The yield of solid product is 2.4 g (60.0 %).

### EXAMPLE: 16

### PREPARATION OF N-[[4-(5-METHYL-3-PHENYL-4-ISOXAZOLYL) PHENYL] SULFONYL] PROPANAMIDE (Parecoxib, Ib):

4-(5-methyl-3-phenyl-4-isoxazolyl) benzenesulfonamide (25.0 g) and propionic anhydride (100 mL) were charged to the clean and dry round bottom flask and heated to 50 °C. Sulfuric acid (100.00ml) was added slowly and the mixture warmed to 55.5°C. within a 10 minute period after the addition was completed. The reaction mixture was then heated to 80°C and held for approximately 10 minutes. Heating was discontinued, and the mixture was allowed to cool to 25-35°C.Ice water was charged into another round bottom flask and was slowly cooled to 0-5 °C followed by stirring at 0-5°C for 30-45 minutes. The solid was filtered and washed with water 2-butanol and suck dried perfectly. The wet solid was charged into another round bottom flask followed by acetone and stirred for 10-15 minute till the clear solution. DM water (81 mL) was added to the reaction mixture at 25-35°C and stirred at 25-35°C for 30-45 minutes. The resultant solid was filtered and washed with DM water (1250 mL) followed by petroleum ether. The solid was further dried in a vacuum at 60°C to give the titled solid product (20.5 g 69.6% yield).

### EXAMPLE: 17

### PREPARATION OF N - [[4-(5-METHYL-3-PHENYL-4-ISOXAZOYL) PHENYL] SULFONYL] PROPANAMIDE, SODIUM SALT (Parecoxib Sodium)

N-[[4-(5-methyl-3-phenyl-4-isoxazolyl)phenyl]-sulfonyl]propanamide (10.0 g) and 150 ml of absolute ethanol were charged to clean and dried round bottom flask. The slurry was heated to 45°C and held for 30 minutes and a solution of approximately 1.17gm sodium hydroxide in ethanol (50.0mL) was added to the reaction vessel at 45°C for 30 minutes. The mixture was slowly cooled to 0-5°C. and held for about 60-90 min. The solid was collected by filtration. The wet cake was washed twice with two 30-mL portions of absolute ethanol and was pulled dry under vacuum to get titled compound (7.5gm, 70.8%).

## Claims

1. A process for preparing a compound of Formula IX 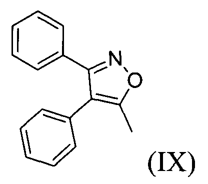 comprising
(a) reacting a compound of Formula V 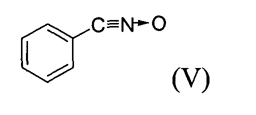 with a compound of Formula VII 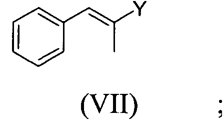 and
(b) removing said Y group from the product of Step (a),
wherein Y is

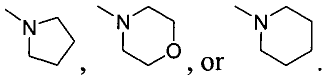

2. The process of claim 1, wherein said Y is

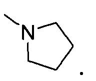

3. The process of claim 1, wherein said Y is

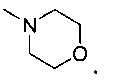

4. The process of claim 1, wherein said Y is

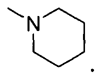

5. A process for preparing a compound of Formula I 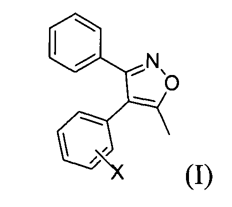 comprising
(a) reacting a compound of Formula V 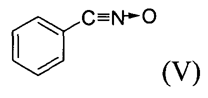 with a compound of Formula VII 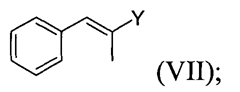
(b) removing Y from the product of Step (a);
(c) reacting ClSO₃H with the product of Step (b); and
(d) substituting the chloro group of the product of Step (c) with R to afford the compound of Formula I
wherein X is R-SO₂-, where R is C₁-C₆ alkyl, C₁-C₆ alkanoylamino and amino; and
Y is

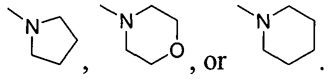

6. The process of claim 5, wherein said R is -NH₂.

7. The process of claim 6, wherein said -NH₂ group is at the para position.

8. The process of claim 5, wherein said R is propanamidyl group.

9. The process of claim 8, wherein said -NH₂ group is at the para position.

10. The process of claim 5, wherein said reaction of Step (a) is done in dichloromethane.

11. The process of claim 5, wherein said Y is

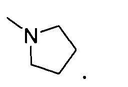

12. The process of claim 5, wherein said Y is

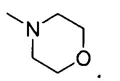

13. The process of claim 5, wherein said Y is

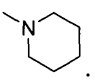

14. The process of claim 5, wherein said substitution is done by amination using NH₂OH.

15. A process for preparing valdecoxib comprising
(a) reacting a compound of Formula V 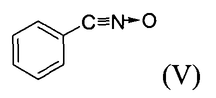 with a compound of Formula VII 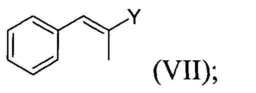
(b) removing Y from the product of Step (a);
(c) reacting ClSO₃H with the product of Step (b); and
(d) substituting the chloro group of the product of Step (c) with amino group to afford said valdecoxib
where Y is

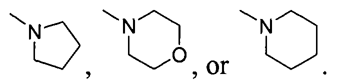

16. The process of claim 15, which further comprises
(a) reacting the valdecoxib with a propionating agent.

17. The process of claim 16, wherein said propionating agent is propionic anhydride.

18. A process for preparing parecoxib comprising
(a) a) reacting a compound of Formula V 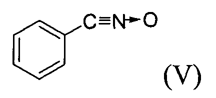 with a compound of Formula VII 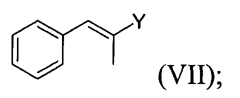
(b) removing Y from the product of Step (a);
(c) reacting ClSO₃H with the product of Step (b); and
(d) substituting the chloro group of the product of Step (c) with propanamide to afford said parecoxib
where Y is

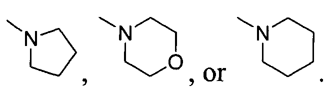

19. The process of claim 18, wherein said Y is

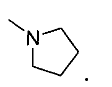

20. The process of claim 18, wherein said Y is

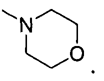

21. The process of claim 18, wherein said Y is
